(19) Europäisches Patentamt
European Patent Office
Office européen des brevets

(11) Veröffentlichungsnummer: **0 153 476**
**B1**

(12) # EUROPÄISCHE PATENTSCHRIFT

(45) Veröffentlichungstag der Patentschrift: **15.10.86**

(51) Int. Cl.⁴: **C 07 C 103/76**, C 07 C 102/00

(21) Anmeldenummer: **84115522.9**

(22) Anmeldetag: **15.12.84**

(54) **Verfahren zur Herstellung von Hydroxyphenylessigsäureamiden.**

(30) Priorität: **18.01.84 DE 3401503**

(43) Veröffentlichungstag der Anmeldung: **04.09.85 Patentblatt 85/36**

(45) Bekanntmachung des Hinweises auf die Patenterteilung: **15.10.86 Patentblatt 86/42**

(84) Benannte Vertragsstaaten: **BE CH DE FR GB LI**

(56) Entgegenhaltungen:
**GB-A-1 154 189**
**US-A-4 128 554**

(73) Patentinhaber: **BASF Aktiengesellschaft, Carl-Bosch- Strasse 38, D-6700 Ludwigshafen (DE)**

(72) Erfinder: **Merger, Franz, Dr. Chem., Max- Slevogt-Strasse 25, D-6710 Frankenthal (DE)**
Erfinder: **Kummer, Rudolf, Dr. Chem., Kreuzstrasse 6, D-6710 Frankenthal (DE)**
Erfinder: **Hutmacher, Hans- Martin, Dr. Chem., Ruedigerstrasse 70, D-6700 Ludwigshafen (DE)**

**EP 0 153 476 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein neues Verfahren zur Herstellung von Hydroxyphenylessigsäureamiden durch Umsetzung von N,N-disubstituierten Aminomethylphenolen mit Kohlenmonoxid in Gegenwart von Metallcarbonylen bei einer Temperatur von 60 bis 200°C und einem Druck von 50 bis 500 bar.

Es ist aus GB—A—1 154 189 bekannt, daß man aromatische Lactone mit Aminen in einem inerten Lösungsmittel bei −50 bis +150°C zu o-Hydroxyphenylessigsäureamiden umsetzt.

Man kann (Helv. *45* (1962), Seiten 270 bis 276) ebenfalls w. Tribenzyloxybenzylalkohol mit $SOCl_2$ in das entsprechende Chlorid und anschließend dieses mit Kaliumcyanid in das Benzylnitril überführen. Das Nitril wird dann alkalisch verseift, die gebildete Säure über das Säurechlorid mit Ammoniak, Ethylamin oder Diethylamin zu den entsprechenden Di-bzw. Tribenzyloxyphenylessigsäureamiden umgesetzt, die durch Hydrogenolyse in Gegenwart von Palladiumkohle zu den phenolischen Amiden entbenzyliert werden.

Die DE—A 20 62 956 beschreibt die Umsetzung von 2-Acetoxy-5-(p-fluorophenyl)-benzoylchlorid, das aus der entsprechenden Benzoesäure mit $SOCl_2$ hergestellt wird, mit Diazomethan in Ether zu 4-Acetoxy-3-diazomethylcarbonyl-4'-fluorobiphenyl, dessen Umsetzung mit einem wäßrigen Gemisch von Silberoxid, Kaliumcarbonat und Natriumthiosulfat und Aufarbeitung des Rekationsgemisches (Filtration, Abkühlung, Ansäuern und erneute Filtration des Filtrats, Umkristallisation in Toluol) zu 5-(p-Fluorophenyl)-2-hydroxyphenylessigsäure. Aus dieser Säure kann in üblicher Weise das Amid hergestellt werden.

Es wurde nun gefunden daß man Hydroxyphenylessigsäureamide der Formel I

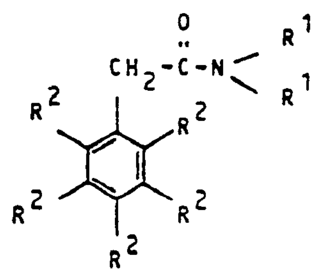

(I),

worin die einzelnen Reste $R^1$ und $R^2$ gleich oder verschieden sind und jeweils einen aliphatischen Rest bedeuten können, mindestens einer der Reste $R^2$ eine Hydroxylgruppe bezeichnet, die übrigen Reste $R^2$ auch jeweils für ein Wasserstoffatom, ein Halogenatom, einen cycloaliphatischen, araliphatischen, aromatischen Rest, eine Alkoxygruppe oder eine Aryloxygruppe stehen können, zwei benachbarte Reste $R^2$ mit den beiden benachbarten Kohlenstoffatomen auch Glieder eines anellierten aromatischen Restes bezeichnen können, die beiden Reste $R^1$ zusammen mit dem benachbarten Stickstoffatom auch Glieder eines heterocyclischen Ringes bedeuten können, vorteilhaft erhält, wenn man N,N-disubstituierte Aminomethylphenole der Formel II

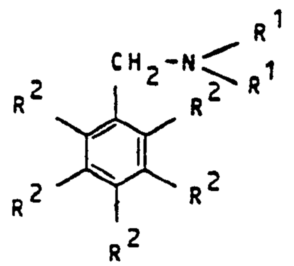

(II),

worin $R^1$ und $R^2$ die vorgenannten Bedeutungen besitzen, mit Kohlenmonoxid in Gegenwart von Metallcarbonylen bei einer Temperatur von 60 bis 200°C und einem Druck von 50 bis 500 bar umsetzt.

Die Umsetzung kann für den Fall der Verwendung von 2-N,N-Dimethylaminomethylphenol durch die folgenden Formeln beschrieben werden:

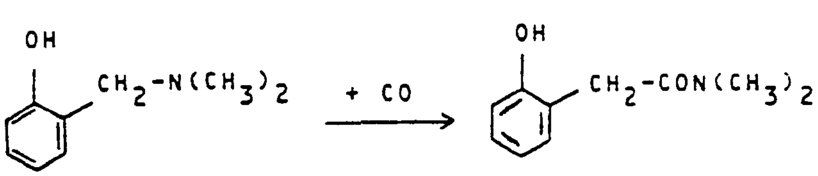


Im Hinblick auf den Stand der Technik liefert das Verfahren nach der Erfindung auf einfacherem und wirtschaftlicherem Wege Hydroxyphenylessigsäureamid in besserer Ausbeute, Raumzeitausbeute und Reinheit. Alle diese Vorteile des erfindungsgemäßen Verfahren sind mit Bezug auf die bekannten Verfahren überraschend.

N,N-disubstituierte Aminomethylphenole II sind in bekannter Weise, z.B. durch Mannichreaktion aus Phenolen, Formaldehyd und sekundären Aminen ohne zusätzliche Hilfsstoffe, zugänglich (Houben-Weyl, Methoden der Organischen Chemie, Band XI/1, Seite 755 bis 759).

Ausgangsstoff II kann mit Kohlenmonoxid in stöchiometrischer Menge oder im Überschuß jeder Komponente zur anderen, zweckmäßig in einem Verhältnis von 1 bis 50, bevorzugt 2 bis 10 Mol Kohlenmonoxid je Mol Ausgangsstoff II, umgesetzt werden. Bevorzugte Ausgangsstoffe II und dementsprechend bevorzugte Endstoffe I sind solche, in deren Formeln die einzelnen Reste $R^1$ und $R^2$ gleich oder vershieden sind und jeweils einen Alkylrest mit 1 bis 8, bevorzugt 1 bis 4 Kohlenstoffatomen bedeuten können, zwei Reste $R^2$ oder insbesondere einen Rest $R^2$, zweckmäßig in 4-Stellung und bevorzugt in 2- und/oder 6-Stellung, jeweils eine Hydroxylgruppe bezeichnen, die übrigen Reste $R^2$ auch jeweils für ein Wasserstoffatom, ein Chloratom, Bromatom, einen Cycloalkylrest mit 5 bis 8 Kohlenstoffatomen, einen Aralkyl- oder Alkylarylrest mit 7 bis 12 Kohlenstoffatomen, einen Phenylrest, ein Alkoxygruppe mit 1 bis 4 Kohlenstoffatomen oder eine Phenoxygruppe stehen können, zwei benachbarte Reste $R^2$ mit den beiden benachbarten Kohlenstoffatomen auch Glieder eines anellierten Benzolringes bezeichnen können, die beiden Reste $R^1$ zusammen mit dem benachbaren Stickstoffatom auch Glieder eines 5- oder 6-gliedrigen heterocyclischen Ringes, der neben dem Stickstoffatom noch ein weiteres Stickstoffatom oder ein Sauerstoffatom enthalten kann, bedeuten können. Die vorgenannten Reste können noch durch unter den Rekationsbedingungen inerte Gruppen, z.B. Alkyl- oder Alkoxygruppen mit jeweils 1 bis 4 Kohlenstoffatomen, substituiert sein.

Es kommen z.B. folgende Ausgangsstoffe II in Betracht: In 2-, 4-, 6- oder 2,6-Stellung durch die N,N-Dimethyl-, N,N-Diethyl-, N,N-Dipropyl-, N,N-Diisopropyl, N,N-Dibutyl, N,N-Diisobutyl, N,N-Di-sek.Butyl-, N,N-Di-tert.-Butyl.-aminomethylgruppe, Piperidino-(N)-methyl, Morpholino-(N)-methyl-gruppe, substituierte Phenole; vorgenannte, je nach Stellung der Aminomethylgruppe in 2-, 3-, 4-, 5- oder 6-Stellung einfach oder in 2,3-, 2,4-, 2,5-, 2,6-, 3,5-, 3,4-Stellung gleich oder unterschiedlich zweifach durch Chloratome, Bromatome, Benzyl-, Phenyl-, Methyl-, Ethyl-, Propyl-, Isopropyl-, Butyl-, Isobutyl-, sek.-Butyl-, Cyclohexyl-, tert.-Butyl-, Methoxy-, Ethoxy-, Propoxy-, Isopropoxy-, Butoxy-, Phenoxy-, sek.-Butoxy-, Isobutoxy-, tert.-Butoxy-Gruppe substituierte 2-, 4- oder 6-Aminomethylphenole; 1-N,N-Dimethylaminomethyl-naphthol-(2); bevorzugt: 2-N,N-Dimethylaminomethylphenol, 2-N,N-Dimethylaminomethyl-4-methylphenol, 2-N,N-Dimethylaminomethyl-4-tert.-butyl-phenol, 2-N,N-Dimethylaminomethyl-4-tert.-octyl-phenol, (2-N,N-Dimethyl-aminomethyl-4-(1,1,2,2-tetramethylbutyl)-phenol, 2-N,N-Dimethyl-aminomethyl-4-chlor-phenol, 2-N,N-Dimethylaminomethyl-4,6-dichlor-phenol, 2,6-Di-tert.-butyl-4-N,N-dimethylaminomethyl-phenol, 2-N,N-Dimethyl-, 2-N,N-Diethyl-, 2-N,N-Dipropyl-, 2-N,N-Diisopropyl-, 2-N,N-Dibutyl-, 2-N,N-Diisobutyl-, 2-N,N-Di-sek.-butyl-, 2-N,N-Di-tert.-butyl-aminomethylphenol.

Die Umsetzung wird bei einer Temperatur von 60 bis 200, vorteilhaft zwischen 60 und 175°C, bevorzugt von 80 bis 170°C, insbesondere von 100 bis 150°C, mit einem Druck von 50 bis 500 bar, bevorzugt 150 bis 300 bar, kontinuierlich oder diskontinuierlich durchgeführt. Zweckmäßig verwendet man unter den Reaktionsbedingungen inerte, organische Lösungsmittel. Als Lösungsmittel kommen z.B. in Frage: Ester wie Methylacetat, n-Propylacetat, Methylpropionat, Butylacetat, Ethylformiat, Essigester, Phenylacetat; Ether, z.B. Ethylpropylether, Methyl-tert.-butylether, Diisopropylether, Diethylether, Tetrahydrofuran, Dioxan; Alkanole und Cycloalkanole wie Ethanol, Methanol, n-Butanol, Isobutanol, tert.-Butanol, Glykol, Glycerin, n-Propanol, Isopropanol, Amylalkohol, Cyclohexanol, 2-Methyl-4-pentanon, Ethylenglykolmonoethylether, 2-Ethylhexanol, Methylglykol, n-Hexanol; und entsprechende Gemische. Zweckmäßg verwendet man das Lösungsmittel in einer Menge von 50 bis 10.000 Gewichtsprozen, vorzugsweise von 50 bis 500 Gewichtsprozent, bezogen auf Ausgangsstoff II. Verwendet man Alkohole als Lösungsmittel, erhält man Gemische aus Hydroxyphenylessigsäureamiden I und den dem Alkohol entsprechenden Hydroxyphenylessigsäureestern.

Das nach den üblichen Verfahren hergestellte Kohlenmonoxid ist in reinem und teils schon in rohem Zustand für das erfindungsgemäß Verfahren geeignet. Zum Beispiel kann das aus der Direktsynthese aus Koks und Sauerstoff (Otto-Verfahren); aus der Abtrennung aus Synthesegasen, Generatorgas, Wassergas, Verkokungsgas, Verschwelungsgas, Gasgemischen der Steinkohle-bzw. der Koksvergasung; gewonnene und in üblicher Weise, z.B. mit der Kupferlaugenwäsche, dem Cosorbverfahren, der Flüssig-Methanwäsche, der Tieftemperaturtrennung, aufgearbeitete rohe Kohlenmonoxid verwendet werden. Es kann noch Verunreinigungen wie Kohlendioxid, Wasserstoff, Methan, Stickstoff, Argon enthalten, wobei zweckmäßig die Gesamtmenge der Verunreinigungen an Kohlendioxid, Methan und insbesondere Wasserstoff 3 Gewichtsprozent des Kohlenmonoxids nicht übersteigt. Der erfindungsgemäße höhere Reaktionsdruck wird zweckmäßig durch den Druck des eingeleiteten Kohlenmonoxid zusammen mit dem Eigendruck der übrigen Reaktionsteilnehmer bei der gewählten Reaktionstemperatur eingestellt, gegebenenfalls können zur Druckeinstellung auch unter den Reaktionsbedingungen inerte Gase, z.B. Stickstoff, verwendet werden.

Als Metallcarbonyle kommen reine Carbonyle, Carbonyle, deren Kohlenmonoxid teilweise durch neutrale oder geladene Liganden substituiert ist, und Carbonylwasserstoffe in Frage, zweckmäßig solche des Osmiums, Iridiums, vorteilhaft des Eisens, Nickels, Rutheniums, bevorzugt des Rhodiums und insbesondere bevorzugt solche des Kobalts. Bezüglich der Herstellung der Carbonyle wird auf Ullmanns Encyklopädie der technischen Chemie, Band 12, Seiten 312 bis 324, verwiesen. Ebenfalls kommen anstelle vorgenannter Carbonyle die entsprechenden Metalle oder Metallverbindungen, die unter den

Reaktionsbedingungen solche Carbonyle bilden können, in Betracht. Als Metallverbindungen können z.B. die Halogenide, insbesondere Bromide und Iodide, Oxide, Sulfate, Carbonate, Hydroxyde, Carboxylate, zweckmäßig von Säuren mit 1 bis 4 Kohlenstoffatomen, vorgenannter Metalle verwendet werden. Substituenten der substituierten Carbonyle zind z.B. Trialkylverbindungen, Triarylverbindungen und Trihalogenide des Phosphors, Amine, Isonitrile, Acetylaceton, Halogene, z.B. in Gestalt von Carbonylhalogeniden wie Chloriden oder Iodiden. Zweckmäßig verwendet man 0,005 bis 0,5, vorzugsweise 0,05 bis 0,2 Mol Metallcarbonyl je Mol Ausgangsstoff II.

Es kommen z.B. als Metallcarbonyle in Betracht: $Fe(CO)_5$, $Ni(CO)_4$, $Ru(CO)_5$, $Rh_2(CO)_8$, $OS(CO)_5$, $Ir_2CO)_8$; $((C_6H_5)_3P)_2Ni(CO)_2$, $((C_6H_5)_3P)_2Fe(CO)_4$, $[Ru(CO)_2J_2]_x$, $OS(CO)_4Cl_2$ $Ir(CO)_2Br_2$; $HCo(CO)_4$, $H_2Fe(CO_4)$, bevorzugt Dikobaltoctacarbonyl $Co_2(CO)_8$ oder Rhodiumdicarbonylacetylacetonat $Rh(CO)_2(C_5H_7O_2)$.

Die Reaktion kann wie folgt durchgeführt werden: Ein Gemisch von Ausgangsstoff II, Kohlenmonoxid und dem Metallcarbonyl, gegebenenfalls zusammen mit einem Lösungsmittel, wird bei der Reaktionstemperatur und dem Reaktionsdruck während 1 bis 12 Stunden gehalten. Vorteilhaft wird man in einem Reaktor ein Gemisch aus Ausgangssstoff II, Metallcarbonyl und einem geeigneten Lösungsmittel vorlegen. Nach dem Aufpressen von Kohlenmonoxid bei Raumptemperatur wird die Temperatur bis auf die Reaktionstemperatur gesteigert. Dann wird gegebenenfalls Kohlenmonoxid nachgepreßt, um den Reaktionsdruck zu erreichen. Die Umsetzung wird nun unter den angegebenen Bedingungen durchgeführt, dann das Gemisch abgekühlt und entspannt. Nach der Umsetzung kann man den Katalysator durch Behandeln mit einer verdünnten Säure in Gegenwart eines Oxidationsmittels, z.B. Luft zerstören. Der Endstoff wird nun aus dem Gemisch in üblicher Weise, z.B. durch Destillation und Filtration abgetrennt. Vorteilhaft lassen sich in vielen Fällen die Amide bei Wahl geeigneter Lösungsmittel durch Kristallisation direkt aus dem Reaktionsgemisch abtrennen.

Die nach dem Verfahren der Erfindung erhältlichen Hydroxyphenylessigsäureamide sind wertvolle Ausgangsstoffe für die Herstellung von Pflanzenschutzmitteln, Farbstoffen und Pharmazeutika. Durch saure oder alkalische Verseifung sind aus ihnen leicht in bekannter Weise und mit guten Ausbeuten die freien hydroxyphenylessigsäuren zugänglich, die in bekannter Weise in die in CH—A 589 595 beschriebenen Salze ungewandelt werden. Diese Säuren und Salzer besitzen zum Teil ödem- und entzündungshemmende, fiebersenkende und analgetische Wirkung. Bezüglich der Verwendung wird auf die vorgenannten Veröffentlichungen verweisen.

## Beispiel 1

151 Gramm 2-N,N-Dimethylaminomethylphenol wurden mit 315 Gramm Essigester und 15 Gramm Dicobaltoctacarbonyl in einem Autoklaven bei 120°C 6 Stunden unter einem Druck von 220 bar Kohlenmonoxid gerührt. Nach Abkühlen Auf Raumtemperatur und Entspannen auf Normaldruck wurden aus dem Reaktionsgemisch unter vermindertem Druck 160 Gramm Essigester abdestilliert. Nach Versetzen des Rückstandes mit 200 Teilen Diethylether kristallisierten 121,7 Teile 2-Hydroxyphenylessigsäure-N,N-dimethylamid vom Fp. 92°C aus, während die Mutterlauge noch 31 Gramm unumgesetzten Ausgangsstoff enthielt. Dies entspricht einer Ausbeute an Endstoff von 68% der Theorie, bezogen auf eingesetztes, bzw. 85,6%, bezogen auf umgesetztes 2-N,N-Dimethylaminomethylphenol.

## Beispiel 2

274,5 Gramm 2-N,N-Dimethylaminomethyl-4-methyl-phenol wurden mit 660 Gramm Tetrahydrofuran und 30 Gramm Dicobaltoctacarbonyl in einem Autoklaven 6 Stunden bei 120°C unter einem Druck von 200 bar Kohlenmonoxid geschüttelt. Nach Abkühlen (auf Raumtemperatur) und Entspannen (auf Normaldruck) wurden aus dem reaktionsgemisch unter vermindertem Druck 330 Gramm Tetrahydrofuran abdestilliert und der Rückstand mit 350 Gramm Diethylether versetzt. Dabei kristallisierten 138,7 Gramm 2-Hydroxy-5-methyl-phenylessigsäure-N,N-dimethylamid vom Fp. 125 bis 126°C aus, während die Mutterlauge noch 107 Gramm unumgesetzten Ausgangsstoff enthielt. Dies entspricht einer Ausbeute von 43,2% der Theorie, bezogen auf eingesetztes, bzw. 70,8%, bezogen auf umgesetztes 2-N,N-Dimethylaminomethyl-4-methylphenol.

## Beispiel 3

230 Gramm 2-N,N-Dimethylaminomethyl-4-tert.-octyl-phenol wurden mit 360 Gramm Essigester und 30 Gramm Dicobaltoctacarbonyl 6 Stunden bei 120°C unter einem Druck von 200 bar Kohlenmonoxid geschüttelt. Nach Abkühlen und Entspannen erhielt man durch Kristallisation aus dem Reaktionsgemisch 174,1 Gramm 2-Hydroxy-5-tert.-octyl-phenylessigsäure-N,N-dimethylamid vom Fp. 156 bis 157°C, während die Mutterlauge noch 57,5 Gramm unumgesetzten Ausgangsstoff enthielt. Dies entspricht einer Ausbeute von 68,4% der Theorie, bezogen auf eingesetztes, bzw. 91,2%, bezogen auf umgesetztes 2-N,N-Dimethylaminomethyl-4-tert.-octylphenol.

## Beispiel 4

2-N,N-Dimethylaminomethyl-4,6-dichlor-phenol wurde analog Beispiel 1 in methanolischer Lösung (315 g Methanol) carbonyliert. Man erhielt ein Gemisch (1 Mol Amid zu 3 Mol Ester) aus 2-Hydroxy-3,5-dichlor-phenylessigsäure-N,N-dimethylamid vom Fp. 173°C und 2-Hydroxy-3,5-dichlor-phenylessigsäuremethylester vom Fp. 103 bis 104°C. Die Verseifung beider Komponenten auf übliche Weise ergab 2-Hydroxy-3,5-dichlor-phenylessigsäure vom Fp. 138 bis 139°C (Säurezahl 252) in einer Ausbeute von 85% der Theorie.

Beispiel 5

Analog Beispiel 1 wurde 2-N,N-Dimethylaminomethyl-4,6-dichlor-phenol mit Essigester als Lösungsmittel umgesetzt. Man erhielt 2-Hydroxy-3,5-dichlor-phenylessigsäure-N,N-dimethylamid vom Fp. 173°C in einer Ausbeute von 80% der Theorie.

**Patentanspruch**

Verfahren zur Herstellung von Hydroxyphenylessigsäureamiden der Formel I

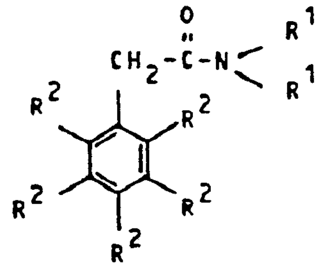

(I),

worin die einzelnen Reste $R^1$ und $R^2$ gleich oder verschieden sind und jeweils einen aliphatischen Rest bedeuten können, mindestens einer der Reste $R^2$ eine Hydroxylgruppe bezeichnet, die übrigen Reste $R^2$ auch jeweils für ein Wasserstoffatom, ein Halogenatom, einen cycloaliphatischen, araliphatischen, aromatischen Rest, eine Alkoxygruppe oder eine Aryloxygruppe stehen können, zwei benachbarte Reste $R^2$ mit den beiden benachbarten Kohlenstoffatomen auch Glieder eines anellierten aromatischen Restes bezeichnen können, die beiden Reste $R^1$ zusammen mit dem benachbarten Stickstoffatom auch Glieder eines heterocyclischen Ringes bedeuten können, dadurch gekennzeichnet, daß man N,N-disubstituierte Aminomethylphenole der Formel II

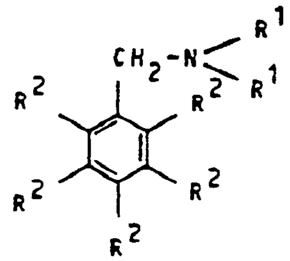

(II),

worin $R^1$ und $R^2$ die vorgenannten Bedeutungen besitzen, mit Kohlenmonoxid in Gegenwart von Metallcarbonylen bei einer Temperatur von 60 bis 200°C und einem Druck von 50 bis 500 bar umsetzt.

**Revendication**

Procédé de préparation d'amides d'acides hydroxyphénylacétiques de formule I

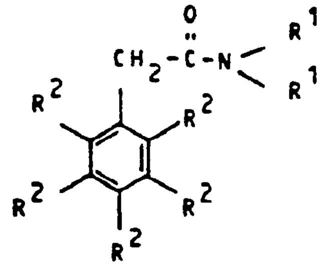

(I),

dans laquelle les différents radicaux $R^1$ et $R^2$ sont identiques ou dissemblables et peuvent représenter chacun un radical aliphatique, l'un au moins des symboles $R^2$ représente un groupement hydroxyle, les autres symboles $R^2$ peuvent être mis chacun pour un atome d'hydrogène, un atome d'halogène, un radical cycloaliphatique, araliphatique, aromatique, un groupement alcoxy ou un groupement aryloxy, deux radicaux $R^2$ voisins puivant aussi représenter, avec les deux atomes de carbone voisins, des maillons d'un radical aromatique condensé, les deux radicaux $R^1$ pouvant aussi former, avec l'atome d'azote voisin, des maillons d'un noyau hétérocyclique, caractérisé en ce qu'on fait réagir des aminométhylphénols N,N-bisubstitués de formule II

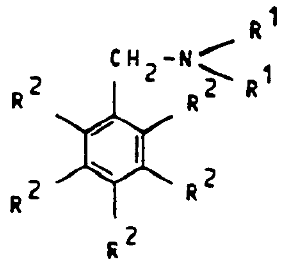

(II),

dans laquelle $R^1$ et $R^2$ ont les significations données ci-dessus, avec l'oxyde de carbone en présence de métal-carbonyles à une température de 60 à 200°C et sous une pression de 50 à 500 bars.

**Claim**

A process for the preparation of a hydroxyphenylacetamide of the formula I

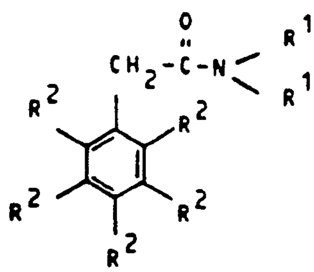

(I),

where the individual radicals $R^1$ and $R^2$ are identical or different and each may denote an aliphatic radical; at least one of the radicals $R^2$ is hydroxyl, and the other radicals $R^2$ may also each denote hydrogen, halogen, a cycloaliphatic, araliphatic or aromatic radical, alkoxy or aryloxy; two adjacent radicals $R^2$ with the two adjacent carbon atoms may also be members of a fused aromatic radical; and the two radicals $R^1$ together with the adjacent nitrogen atom may also be members of a heterocyclic ring, wherein an N,N-disubstituted aminomethylphenol of the formula II

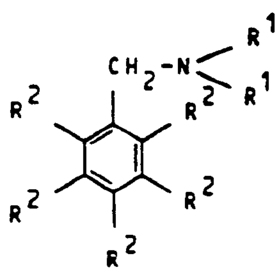

(II),

where $R^1$ and $R^2$ have the above meanings, is reacted with carbon monoxide in the presence of a metal carbonyl at a temperature of from 60 to 200°C and under a pressure of from 50 to 500 bars.